Europäisches Patentamt

European Patent Office

Office européen des brevets

(11) Veröffentlichungsnummer : **0 108 360 B2**

# NEUE EUROPÄISCHE PATENTSCHRIFT

(45) Veröffentlichungstag der neuen Patentschrift :
03.04.91 Patentblatt 91/14

(51) Int. Cl.⁵ : **A61N 1/362**

(21) Anmeldenummer : 83110823.8

(22) Anmeldetag : 28.10.83

(54) Herzschrittmacher zum Beenden einer Tachykardie.

(30) Priorität : 02.11.82 DE 3240430

(43) Veröffentlichungstag der Anmeldung :
16.05.84 Patentblatt 84/20

(45) Bekanntmachung des Hinweises auf die
Patenterteilung :
16.09.87 Patentblatt 87/38

(45) Bekanntmachung des Hinweises auf die
Entscheidung über den Einspruch :
03.04.91 Patentblatt 91/14

(84) Benannte Vertragsstaaten :
DE FR NL

(56) Entgegenhaltungen :
DE-A- 3 110 013
GB-A- 2 076 655
US-A- 4 222 385
US-A- 4 280 502
Taschenbuch der Nachrichtenverarbeitung, K.
Steinbuch, Springer Verlag Berlin, 1967,S.1394
und 1395

(73) Patentinhaber : Siemens Aktiengesellschaft
Wittelsbacherplatz 2
W-8000 München 2 (DE)

(72) Erfinder : Elmqvist, Hakan, Dr.
Sunnedalsvaegen 7
S-161 38 Bromma (SE)
Erfinder : Strandberg, Hans
Landsvaegen 51
S-172 36 Sundbyberg (SE)

EP 0 108 360 B2

## Beschreibung

Die Erfindung betrifft einen Herzschrittmacher zum Beenden einer Tachykardie.

Als Tachykardie bezeichnet man die unnatürlich überhöhte Frequenz des Herzschlages, beispielsweise wenn das Herz mehr als 180 Schläge pro Minute macht. Es ist bekannt, dass dieser schnelle Herzrhythmus, wenn er durch retrograde Überleitung verursacht ist, durch elektrische Stimulierungsimpulse gestoppt werden kann. Voraussetzung dafür ist jedoch, dass ein Stimulierungsimpuls innerhalb eines kritischen Zeitintervalles nach einem Tachykardie-Herzschlag, einem sog. Fenster, an das Herz abgegeben wird. Dieses Fenster kann direkt nach der auf einen Herzschlag folgender Refraktärzeit des Herzens oder auch erst unmittelbar vor einem nächsten Herzschlag liegen. Aus Untersuchungen ist es weiter bekannt, dass einzelne Stimulierungsimpulse nicht immer ausreichen, um eine Tachykardie effektiv zu beenden, sondern dass dazu aus mehreren Stimulierungsimpulsen bestehende Sequenzen notwendig sein können. Dabei kann es erforderlich sein, dass die Intervalle zwischen den einzelnen Impulsen einer Sequenz unterschiedlich lang sind. Weiterhin haben Untersuchungen gezeigt, dass die effektiven Sequenzen von der Körperlage eines Patienten, physischen Aktivitäten, Drogen und ähnlichem abhängen können. Ausserdem können sie stark von Patient zu Patient variieren. Bereits für einen Patienten ist es aus den besagten Gründen nicht möglich, eine feste Sequenz zu ermitteln, die stets eine Tachykardie sicher beenden kann.

Es sind bereits eine Reihe von Verfahren bzw. Herzschrittmacher bekannt, die sich damit beschäftigen, wie der richtige Zeitpunkt für die Stimulierung gefunden werden kann. So zeigt die US-PS 3 942 534 ein Verfahren, bei dem das Zeitintervall zwischen der Beendigung der Refraktärzeit und dem nächstfolgenden Tachykardie-Herzschlag in äquidistanten Schritten abgerastert wird. Da das Fenster sehr schmal sein kann, müssen die Schritte sehr klein gewählt werden, so dass das Überstreichen des möglichen Bereiches unter Umständen sehr lange dauern kann.

Aus der US-PS 4 312 356 ist ein Herzschrittmacher bekannt, bei dem die Reaktion des Herzes auf einen Stimulierungsimpuls erfasst und zum Steuern des Intervalles bis zum nächsten Stimulierungsimpuls verwendet wird derart, dass die Veränderung des Intervalls jeweils in Richtung auf die zum Beenden einer Tachykardie effektive Intervallgrösse geschieht.

Aus der US-PS 4 280 502 schliesslich ist ein Verfahren und ein Herzschrittmacher bekannt, bei dem ebenfalls beim Auftreten einer Tachykardie das Intervall zwischen dem Ende der Refraktärzeit und dem nächsten Tachykardie-Herzschlag abgerastert wird, bis eine effektive Stimulierung zum Beenden der Tachykardie gefunden ist. Zusätzlich kann vorab auf ähnliche Weise die Refraktärzeit bestimmt werden. Vorteil dieses bekannten Verfahren und Herzschrittmachers ist es, das der einmal gefundene Wert in einem Register gespeichert und beim erneuten Auftreten einer Tachykardie als erster Versuchswert zum Beenden derselben verwendet wird.

Aus der GB-A-2 076 655 ist ein Verfahren bekannt, bei dem eine Sequenz von zwei Stimulierungsimpulsen zum Beenden einer Tachykardie verwendet wird, wobei die zeitliche Lage dieser Impulse so variiert werden kann, dass der durch die Zeiten beider Impulse aufgespannte zweidimensionale Raum nach und nach abgerastert wird. Nach jeder Sequenz wird die Reaktion des Herzens überwacht und nur, wenn weiterhin eine Tachykardie vorliegt, erneut mit einer veränderten Sequenz stimuliert. Hat eine Sequenz die Tachykardie effektiv beendet, so wird sie in einem Register gespeichert und beim erneuten Auftreten einer Tachykardie als erste Sequenz verwendet. Da die Räume (im eindimensionalen Fall die Fenster), in denen eine Sequenz effektiv ist, von den äusseren Bedingungen abhängen, kann das Abrastern in der Nähe der letzten gespeicherten Sequenz das Auffinden der neuen effektiven Sequenz unter Umständen störend verlängern.

Der vorliegenden Erfindung liegt die Aufgabe zu Grunde, einen Herzschrittmacher anzugeben, mit dem für jeden beliebigen Patienten und unabhängig von den äusseren Bedingungen, denen ein Patient jeweils unterworfen ist, eine auftretende Tachykardie auf einfache und sichere Weise möglichst rasch beendet werden kann.

Diese Aufgabe wird durch die im Anspruch 1 angegebenen Merkmale gelöst. Die Erfindung geht dabei von der Erkenntnis aus, dass zum Beenden einer Tachykardie eine Sequenz von $n$ Stimulierungsimpulsen notwendig ist, wobei jeder der Stimulierungsimpulse dieser Sequenz zu einem bestimmten Zeitpunkt an das Herz abgegeben werden muss. Als Grenzfall der Sequenz soll hierbei auch verstanden werden, dass $n$ den Wert 1 annimmt, d.h. dass die Sequenz eigentlich nur aus einzelnen Impulsen besteht.

Wie Untersuchungen gezeigt haben, bilden die effektiven Sequenzen Bereiche in einem durch die möglichen Zeiten der einzelnen Intervalle der Sequenzen aufgespannten $n$-dimensionalen Raum. Bei $n = 1$, d.h. einzelnen Impulsen, bestehen diese Bereiche aus den bekannten Fenstern.

Bei dem erfindungsgemässen Herzschrittmacher ist nun vorgesehen, dass beim ersten Auftreten einer Tachykardie der $n$-dimensionale Raum in einer der bekannten Weise abgerastert wird, bis eine effektive Sequenz zum Beenden der Tachykardie gefunden ist, und dass diese effektive Sequenz in einem Register eines Speichers abgespeichert wird. Beim erneuten Auftreten einer Tachykardie werden zunächst diese abgespeicherten Werte abgefragt und als erste Sequenz an das Herz abgegeben. Sollte sich diese Sequenz als ineffektiv erweisen, wird erneut die Routine zum Auffinden einer effektiven Sequenz gestartet und der so ermittelte Wert in ein weiteres Register des Speichers eingegeben. Auf diese Art und Weise werden im Laufe der Zeit sämtliche Register des Speichers mit Sequenzen aufgefüllt, die zumindest einmal zum Beenden einer Tachykardie effektiv waren.

Ebenso ist die Möglichkeit vorgesehen, dass von Anfang an ausgewählte Sequenzen in den Registern gespeichert sind und dass zunächst mit diesen Sequenzen stimuliert wird.

Für jede erneute auftretende Tachykardie werden zunächst die gespeicherten Sequenzen ausprobiert um zu sehen, ob eine dieser Stimulierungen bereits erfolgreich die Tachykardie beenden kann. Die Erfindung geht dabei von der Erkenntnis aus, dass es um so leichter ist, eine Tachykardie zu beenden, je wenige Versuche dazu unternommen werden müssen, d.h. je schneller die effektive Sequenz gefunden wird. Durch die Vielzahl der in den Registern gespeicherten Sequenzen, die alle unter unterschiedlichen äusseren Bedingungen zustande gekommen sein können, wird die Wahrscheinlichkeit stark erhöht, das bereits eine dieser gespeicherten Werte effektiv ist. Sollten sich alle Registerwerte als ineffektiv erweisen, so wird eine neue Routine zum Auffinden einer effektiven Sequenz gestartet und dieser Wert in eins der vorhandenen Register eingegeben. Der ursprünglich vorhandene Wert wird dabei gelöscht.

In vorteilhafter Weiterbildung des Herzschrittmachers ist vorgesehen, dass die vorhandenen Registerwerte in einer Weise ausgenützt werden, dass die effektive Sequenz möglichst noch schneller aufgefunden wird. Dazu sind verschiedene Alternativen möglich. So können die Sequenzen beispielsweise in der Reihenfolge, in der sie aufgetreten sind, gelagert und-/oder ausgenutzt werden.

Ein Parameter für das Auftreten unterschiedlicher Sequenzen kann beispielsweise auch die Länge des Tachykardieintervalles sein. Die gefundenen Sequenzen können daher nach den ihnen zugehörigen Tachykardieintervallen (RR-Intervallen) geordnet sein und danach ausgenutzt werden, wie nahe das Intervall einer erneuten Tachykardie an die gespeicherten Tachykardieintervalle herankommt. Eine weitere oder auch zusätzliche Möglichkeit besteht darin, jedem Register eine Gütezahl zuzuordnen, die davon abhängt, wie oft eine in dem entsprechenden Register vorhandene Sequenz effektiv oder ineffektiv war.

Zur Verminderung des Speicherbedarfs ist in einer vorteilhaften Weiterbildung des Herzschrittmachers vorgesehen, dass nur ein Intervall jeder Sequenz in den Registern gespeichert und wiederholt verwendet wird. Damit ergeben sich Sequenzen mit äquidistanten Intervallen. Weiterhin kann auch die Zeit zum Abrastern des n-dimensionalen Raumes zum Auffinden einer effektiven Sequenz erheblich vermindert werden, wenn sich dieses Abrastern auf Sequenzen mit bestimmten Intervallen beschränkt. Bei gleichen Intervallen verkürzt sich die Zeit damit auf einen Wert, der dem Wert der n-ten Wurzel aus der ansonsten benötigten Zeit entspricht.

Der Herzschrittmacher gemäss der vorliegenden Erfindung besteht aus einer herkömmlichen Herzschrittmacher-Elektronik, wie sie beispielsweise für einen inhibierten oder synchronisierten Herzschrittmacher üblich ist und auf die im Rahmen dieser Erfindung nicht näher eingegangen zu werden braucht, sowie einem Tachykardiedetektor, der von der Herzschrittmacher-Elektronik Signale empfängt, wenn ein Herzsignal vorliegt oder beispielsweise ein Stimulierungsimpuls detektiert wurde. Weiterhin enthält der Herzschrittmacher einen Speicher mit N Registern, in die jeweils mindestens die Sequenz von n Stimulierungsimpulsen eingebbar ist und dessen Registerwerte zum Einstellen des Abgabezeitpunktes der von der bekannten Herzschrittmacher-Elektronik erzeugten Stimulierungsimpulse dient. Eine durch den Tachykardiedetektor getaktete Steuerlogik wählt dabei jeweils ein Register aus und/ oder sorgt für eine Änderung der gespeicherten Werte in diesem Register.

Weitere Vorteile der Erfindung ergeben sich aus den Unteransprüchen.

Im folgenden wird die Erfindung anhand von 4 Figuren näher erläutert und beschrieben. Dabei zeigt

Figur 1 in einem ersten Blockschaltbild den prinzipiellen Aufbau eines Herzschrittmachers gemäss der Erfindung,

Die Fig. 2a, b zwei zeitliche Vorläufe verschiedener Signale, die beispielsweise mittels eines EKG-Gerätes registriert werden können,

Figur 3 in einem zweidimensionalen Diagramm Bereiche effektiver Sequenzen und

Figur 4 in einem weiteren Blockschaltbild eine spezielle Ausführungsform des erfindungsgemässen Herzschrittmachers mit Ausnahme der traditionellen Herzschrittmacher-Elektronik.

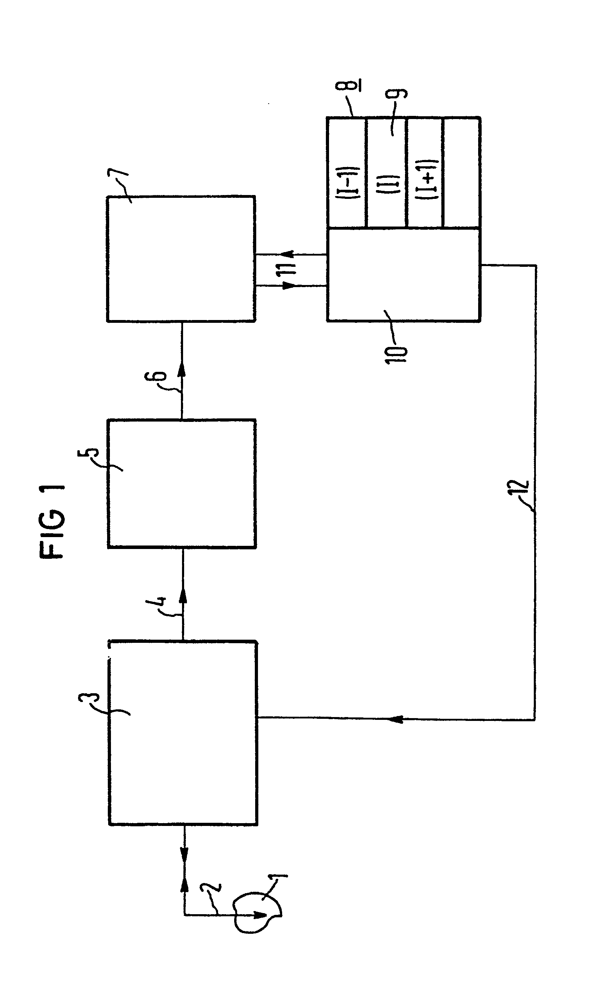

In Figur 1 ist mit 1 schematisch ein Herz dargestellt, in das eine Elektrode 2 eingeführt ist. Für die vorliegende Erfindung ist es dabei unwesentlich, ob ein und dieselbe Elektrode für die Zuführung von Stimulierungsimpulsen und für das Abfühlen von Herzpotentialen verwendet wird oder ob dazu eine bipolare Elektrode oder auch mehrere einzelne Elektroden verwendet werden. Ebenso ist es für diese Erfindung unwesentlich, wo die Stimulierung und wo das Abfühlen im Herzen geschieht. Die Elektrode 2 ist an eine herkömmliche Herzschrittmacher-Elektronik angeschlossen, die durch den Block 3 angedeutet ist und die beispielsweise einen Herzsignaldetektor, einen Stimulierungsimpuls-Generator sowie eine Messeinrichtung zur Bestimmung verschiedener Zeitintervalle enthält. Die Herzschrittmacher-Elektronik 3 erzeugt ein Ausgangssignal auf einer Leitung 4, wenn die Elektrode 2 eine Herztätigkeit oder einen Stimulierungsimpuls registriert hat. Dieses Signal gelangt auf einen Tachykardiedetektor 5, der beispielsweise die Frequenz der empfangenen Signale ermittelt und je nach der Höhe dieser Frequenz festgelegt, ob eine Tachykardie vorliegt oder nicht. Das Ausgangssignal dieses Tachykardiedetektors startet oder stoppt über eine Leitung 6 eine Steuerlogik 7, über die ein Speicher 8 mit einer Anzahl von Registern 9 angesteuert wird. Über die Steuerlogik wird die Wahl des jeweiligen Registers festgelegt und weiterhin bestimmt, ob die in den Registern gespeicherten Daten verändert werden sollen. Der Speicher 8 soll weiterhin eine Registerlogik 10 zum Auslesen respektive Verändern der einzelnen Registerwerte enthalten. Das Ausgangssignal des Speichers 8 wird über eine Leitung 11 auf die Steuerlogik rückgekoppelt. Über eine Leitung 12 wird dieses Ausgangssignal weiterhin als Steuersignal auf die herkömmliche Herzschrittmacher Elektronik 3 zur Steuerung der Stimulationszeiten in Abhängigkeit von den Herzereignissen gegeben. Unter Leitung sollen dabei einzelne Leiter, Leitergruppen und auch Datenbusleitungen verstanden werden.

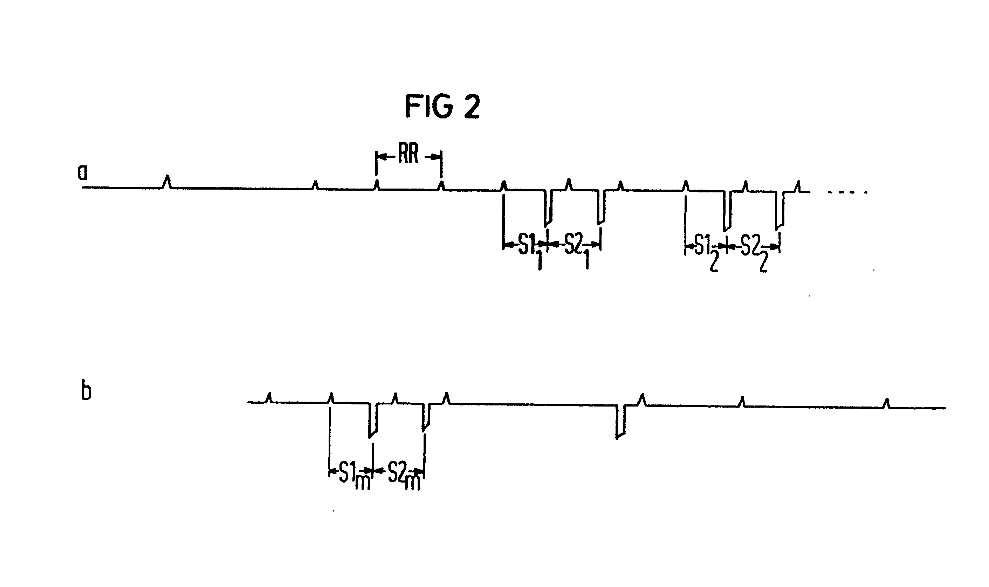

Anhand der Figur 2 wird der zeitliche Verlauf der von der Elektrode 2 registrierten Signale beispielhaft näher beschrieben und dazu genutzt, das Verfahren zum Beenden einer Tachykardie näher zu erläutern. Im Teil a der Figur 2 sind in einem willkürlichen, zeitlichen Massstab als positive Zacken natürliche Herzaktivitäten und als negative Impulse Stimulierungsimpulse dargestellt. Die ersten beiden natürlichen Herzschläge liegen mit so grossem Abstand zueinander, dass eine normale Herzfrequenz vorliegt. Der zeitliche Abstand der nachfolgenden Zacken ist so klein, dass sich das Herz in einem Tachykardiezustand befindet. Das Intervall zwischen zwei Tachykardie-Herzschlägen ist mit RR bezeichnet.

Beispielsweise ist im Teil a der Figur 2 angedeutet, dass nach dem vierten Tachykardie-Herzschlag eine erste Sequenz von Stimulierungsimpulsen an das Herz abgegeben wird, um die Tachykardie zu beenden. Der erste Stimulierungsimpuls liegt dabei eine Zeit $S1_1$ nach dem letzten Tachykardie-Herzschlag. Der zweite Stimulierungsimpuls dieser Sequenz liegt eine Zeitspanne $S2_1$ nach diesem ersten Stimulierungsimpuls. Beispielhaft sind hier Sequenzen mit zwei Stimulierungsimipulsen dargestellt. Es sei aber ausdrücklich darauf hingewiesen, dass unter Umständen bereits einzelne Stimulierungsimpulse eine Tachykardie erfolgreich beenden können oder das es auch notwendig sein kann, Sequenzen mit mehr als zwei Stimulierungsimpulsen zu verwenden.

An diese erste Sequenz schkiesst sich zunächst ein Kontrollintervall an, in dem keine weitere Stimulierung vorgenommen wird. Dieses Intervall besteht im vorliegenden Beispiel aus den an den letzten Stimulierungsimpuls der ersten Sequenz anschliessenden zwei registrierten Herzschlagsignalen. Liegt, wie im vorliegenden Beispiel, weiterhin eine Tachykardie vor, so wird erneut stimuliert, diesmal jedoch mit einer anderen Sequenz $S1_2$, $S2_2$.

Dieses Verfahren wird so lange fortgesetzt, bis eine effektive Sequenz zum Beenden der Tachykardie gefunden ist. Das ist im Teil b der Figur 2 dargestellt. Das Herz wird in diesem Fall mit einer Sequenz $S1_m$, $S2_m$ stimuliert. Im anschliessenden Kontrollintervall tritt überhaupt kein natürlicher Herzschlag auf, so dass nach einer vorbestimmten Zeit die herkömmliche Herzschrittmacher- Elektronik einen einzelnen Stimulierungsimpuls an das Herz abgibt. Die letzte Zacke dieser zeitlichen Darstellung zeigt, dass das Herz wieder auf eine natürliche Herzschlagfolge zurückgekehrt ist.

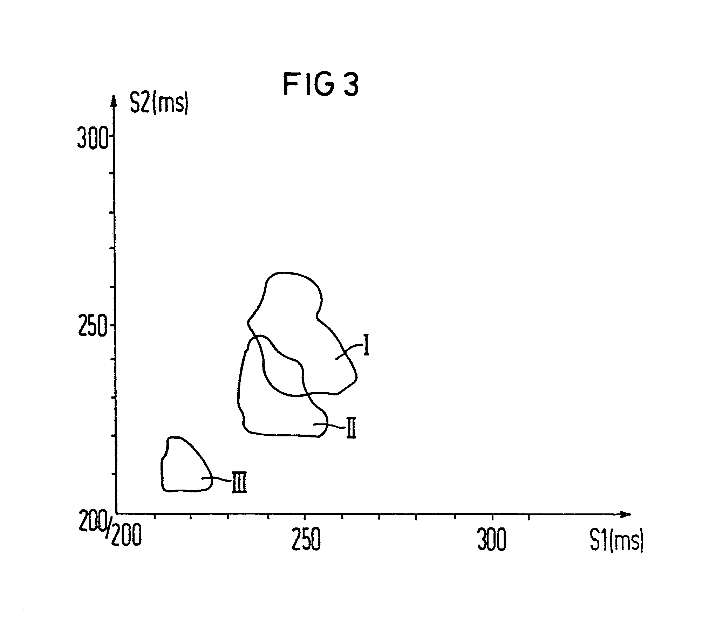

In Figur 3 sind beispielhaft effektive Sequenzen zum Beenden einer Tachykardie dargestellt. Die unterschiedlichen Bereiche I, II und III beruhen dabei auf unterschiedlichen äusseren Bedingungen für den Patienten. So kann der Bereich I für einen ruhenden liegenden Patienten gelten, der Bereich II für einen sitzenden Patienten und der Bereich III beispielsweise für einen Patienten unter Belastung. Die Bereiche können sich teilweise überlappen, machen es aber nicht für alle Bereiche und nicht für alle Patienten. In dem Diagramm gemäss Figur 3 ist auf der Abszisse das Zeitintervall S1 und auf der Ordinate das Zeitintervall S2 in ms aufgetragen. Es sei hier noch einmal darauf hingewiesen, dass die Zeit S1 vom dävorliegenden Tachykardie-Herzschlag aus gemessen wird, die Zeit S2 jedoch vom davorliegenden Stimulierungsimpuls aus.

Es versteht sich von selbst, dass die Zeiten auch auf andere Weise definiert werden können.

Weiterhin ist in der Darstellung gemäss Figur 3 der Koordinatenschnittpunkt bei jeweils 200 ms gewählt worden, d.h. dass nur ein kleiner Zeitausschnitt des gesamten Zeitbereiches dargestellt ist. Der Einfachheit halber ist hier wiederum nur eine Sequenz mit zwei Stimulierungsimpulsen zur Darstellung herangezogen worden. Bei drei Stimulierungsimpulsen für die Sequenzen ergäbe sich in entsprechender Weise ein räumliches Gebilde.

Wie dieser Figur zu entnehmen ist, kann es unter Umständen recht lange dauern, bis eine effektive Sequenz durch Abrastern des gesamten möglichen Bereiches gefunden ist. Es ist daher eine den Patienten erheblich belastende Situation, wenn diese Abrasterung bei jeder neu auftretenden Tachykardie wiederholt werden muss. Selbst die Speicherung eines einzelnen effektiven Stimulierungswertes und das gezielte Absuchen zunächst in dessen unmittelbarer Nähe ergibt keine wesentliche Verbesserung. Liegt dieser gespeicherte effektive Wert beispielsweise im Bereich III gemäss der Figur 3, und befindet sich der Patient beim erneuten Auftreten einer Tachykardie in einern anderen physischen Zustand, so verlängert das Absuchen lediglich in der Nähe des Bereiches III nur die erfolglose Zeit zum Auffinden einer neuen effektiven Sequenz.

Der erfindungsgemässe Herzschrittmacher hingegen stellt von Anfang an eine Vielzahl effektiver Sequenzen zur Verfügung, die in kurzer Zeit alle durchprobiert werden können. Die Wahrscheinlichkeit, dass eine dieser gespeicherten Sequenzen wiederum effektiv ist, ist dabei recht gross. Wenn man ausserdem noch eine Wertung der einzelnen Sequenzen vornimmt, d.h. diese beispielsweise mit einer Gütezahl versieht, so kann diese Wahrscheinlichkeit noch beträchtlich gesteigert werden. In Figur 3 lässt sich beispielsweise aus dem Überschneiden der beiden Bereiche I und II ableiten, dass eine effektive Sequenz in diesem Schnittbereich bereits für 2 unterschiedliche Situationen, denen der Patient ausgesetzt ist, effektiv ist, so dass derartige Sequenzen mit einer grösseren Wahrscheinlichkeit eine Tachykardie beenden können.

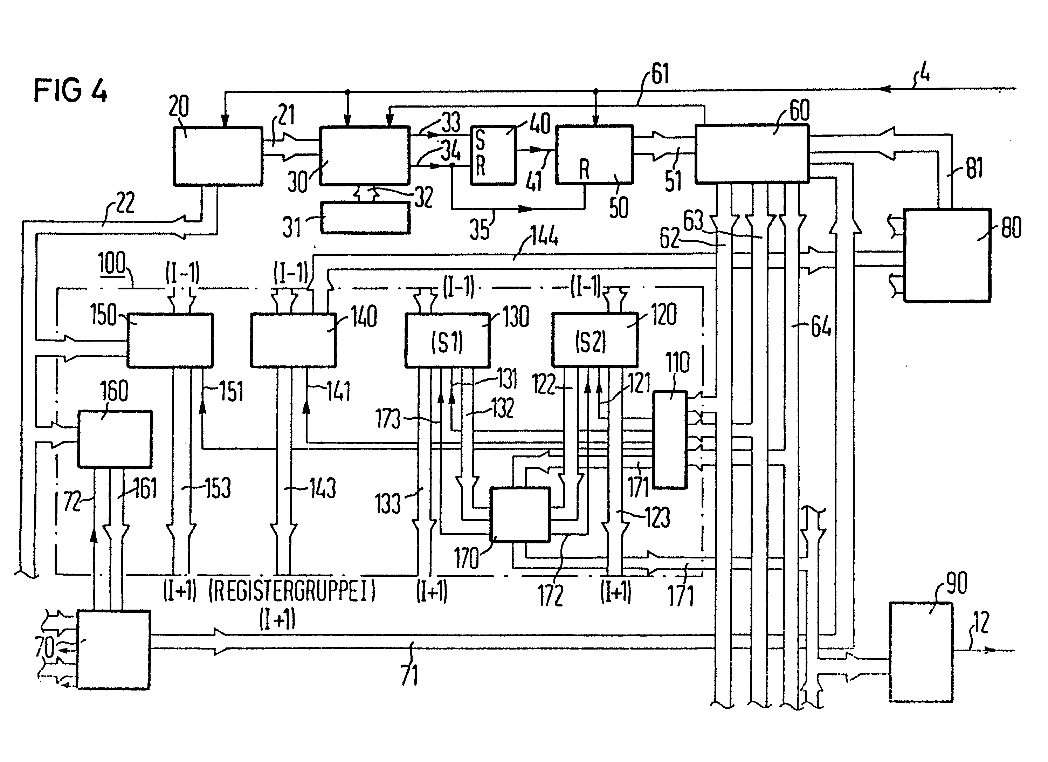

Zur weiteren Erläuterung der Erfindung sind in Figur 4 in einem weiteren Blockschaltbild die wesentlichen neuen Teile des Herzschrittmachers näher beschrieben und erläutert. Die traditionelle Herzschrittmacher-Elektronik 3 gemäss Figur 1 ist der Übersichtlichkeit halber hierbei weggelassen. Die Schnittstelle geht praktisch durch die Leitungen 4 bzw. 12 gemäss Figur 1.

Die über die Leitung 4 von der Herzschrittmacher-Elektronik erhaltenen Signale werden als Rücksetzsignale auf einen getakteten Zähler 20, als Tastimpuls auf einen Digitalkomparator 30 und als Taktsignal auf einen Zähler 50 gegeben. Über eine Datenleitung 21 gelangen die Werte des Zählers 20 ständig auf den Digitalkomparator, dem ausserdem von einem Tachykardie-Register 31 über eine weitere Leitung 32 ein einstellbarer Vergleichswert zugeführt wird. Die Ausgangssignale des Digitalkomparators gelangen über Leitungen 33 bzw. 34 auf den Setz- bzw. Rücksetzeingang eines Speicherelementes 40, dessen Ausgangssignal als Freigabesignal über eine Leitung 41 auf den Zähler 50 gegeben ist. Weiterhin ist das Ausgangssignal des Digitalkomparators über die Leitung 35 auf den Rückstelleingang des Rechners 50 gegeben.

Über eine Datenleitung 51 ist der Rechner 50 mit einem Multiplexer 60 verbunden. Von diesem kann über eine Leitung 61 ein Freigabesignal an den Digitalkomparator 30 abgegeben werden.

Vom Multiplexer 60 gehen weiterhin verschiedene Datenleitungen 62, 63 und 64 zu den einzelnen Registergruppen. Der Übersichtlichkeit halber ist in Figur 4 nur eine einzige Registergruppe 100 dargestellt. Der Aufbau der weiteren Registergruppen ist identisch mit dieser. Die Anzahl N der Registergruppen ist beliebig.

Die Signale des Multiplexers 60 gelangen zunächst auf eine logische Torschaltung 110 und von dort über Leitungen 121, 131, 141, 151 und 171 auf vier Register 120, 130, 140 und 150 sowie einen Multiplexer 170. Im vorliegenden Ausführungsbeispiel enthält jede Registergruppe vier Register, wovon für drei Vorwärts-/Rückwärts-Zähler verwendet werden. Das vierte Register ist ein sog. Latch-Register. Über Datenleitungen 122 und 132 können die in den Zählern 120 bzw. 130 vorhandener, Zählwerte, d.h. die Registerwerte, auf den Multiplexer 170 gegeben werden. Über Datenleitungen 123, 133, 143 und 153 sind die Register der Registergruppe 100 mit den entsprechenden Registern der benachbarten Registergruppen verbunden. Weiterhin enthält jede Registergruppe 100 einen steuerbaren Digitalkomparator 160, dem, ebenso wie dem Latch-Register 150, über eine Datenleitung 22 die Zähldaten des Zählers 20 zugeführt sind. Zusätzlich enthält die Schaltung zwei Prioritätsdekodieren 70 bzw. 80, denen über Leitungen 161 bzw. 144 vom Digitalkomparator 160 bzw. dem Vorwärts-/Rückwärtszähler 140 Signale zugeführt sind. Die Ausgangssignale der Dekodierer 70 bzw. 80 sind über Leitungen 71 bzw. 81 als zusätzliche Steuersignale auf den Multiplexer 60 gegeben.

Vom Prioritätsdekodierer 70 gelangt ausserdem über eine Leitung 72 ein Steuersignal auf den Digitalkomparator 160. Wie ausserdem angedeutet, werden auf die beiden Dekodierer nicht nur Signale der dargestellten Registergruppe 100, sondern auch die entsprechenden Signale der übrigen Registergruppen gegeben. Schliesslich enthält die Schaltung einen einstellbaren Zeitgeber 90, dem die Ausgangssignale des Multiplexers 170 über eine Datenleitung 171 zugeführt Sind.

Die Wirkungsweise der Schaltung ist folgende :

Der Zähler 20 wird von einem hier nicht dargestellten Oszillator getaktet und durch jedes detektierte Herzsignal zurückgesetzt. Wenn der anschliessende Digitalkomparator vom Multiplexer 60 über die Leitung 61 ein Freigabesignal erhalten hat, wird der unmittelbar vor der Rücksetzung des Zählers 20 vorhandene Zählwert mit dem aus dem Tachykardieregister 131 eingelesenen Wert verglichen. Wird dabei eine Tachykardiebedingung festgestellt, so wird das Speicherelement 40 gesetzt und der Zähler 50 freigegeben. Anderenfalls wird das Speicherelement 40 und der Zähler 50 zurückgesetzt.

Der Zähler 50 wird durch die detektierten Herzsignale getaktet und steuert mit seinem Zählwert den Multiplexer. Im vorliegenden Ausführungsbeispiel ist angenommen, dass die Datenleitung 62 für die Auswahl der zu aktivierenden Registergruppe und die Datenleitung 63 zur Ansteuerung der einzelnen Register innerhalb der Registergruppe dient. Die Datenleitung 64 beispielsweise dient zur Ansteuerung des Multiplexers 170.

Für das vorliegende Ausführungsbeispiel ist weiterhin angenommen, dass die Sequenz der Stimulierungsimpulse zwei unterschiedliche Zeitintervalle S1 und S2 aufweist. Das schliesst die Möglichkeit ein, eine Sequenz mit mehreren Zeitintervallen aufzubauen, beispielsweise in der Form S1, S1, S2 oder S1, S2, S1. Es ist nun angenommen, dass der Vorwärts-/Rückwärts-Zähler 120 zur Speicherung des Zeitintervalles S2 und der Vorwärts-/Rückwärts-Zähler 130 zu Speicherung des Zeitintervalles S1 dienen.

Ist über den Multiplexer 60 die Registergruppe 100 ausgewählt, so schaltet der Multiplexer 170 die Registerwerte der Vorwärts-/Rückwärts- Zähler 120 bzw. 130 nacheinander auf den einstellbaren Zeitgeber durch, der damit ein Steuersignal an die übrige Herzschrittmacher-Elektronik zum Einstellen der Stimulationszeiten in Relation zu den Herzereignissen abgibt. Im Digitalkomparator wird anschliessend wiederum kontrolliert, ob die Tachykardiebedingung noch erfüllt ist oder nicht. Liegt weiterhin eine Tachykardie vor, so kann der Multiplexer entweder die nächste Registergruppe ansteuern und der Vorgang wiederholt sich, oder die ursprüngliche Registergruppe kann beibehalten werden und die Zählerinhalte, d.h. die Registerwerte der Zähler 120 bzw. 130, werden verändert. Wie und in welcher Richtung diese Veränderung geschieht, kann über den Multiplexer 170 und Leitung 172 bzw. 173 gesteuert werden. Auf diese Art und Weise können die Registerinhalte so lange geändert werden, bis eine effektive Sequenz zum Beenden einer Tachykardie vorliegt. Nicht expliziet dargestellt ist hier die Möglichkeit, die Register insb. von ausserhalb des Herzschrittmachers auf vorgebbare, insb. programmierbare Werte einzustellen.

Im Latch-Register 150 ist zusätzlich das entsprechende RR-Intervall der Tachykardie gespeichert. Dieses RR-Intervall kann mit dem aktuellen RR-Intervall im Digitalkomparator 160 verglichen werden. Dieser Vergleich wird in sämtlichen Registergruppen entsprechend durchgeführt. Die Signale des Digitalkomparators werden auf einen gemeinsamen Prioritätsdekodierer 70 gegeben, in dem festgestellt wird, welches gespeicherte RR-Intervall dem aktuellen Intervall am nächsten kommt. Der Digitalkomparator kann dabei derart gesteuert werden, dass der Vergleich zunächst grob und danach schrittweise feiner durchgeführt wird. Mit Hilfe dieses Vergleiches und des Prioritätsdekodierers, dessen Signal als Steuersignal auf den Multiplexer 60 gegeben ist, kann erreicht werden, dass die in den einzelnen Registergruppen gespeicherten Sequenzen in einer vom jeweiligen RR-Intervall abhängigen Reihenfolge ausgenutzt werden.

Weiterhin enthält die Schaltung einen Vorwärts-/Rückwärts-Zähler 140 zum Speichern einer Gütezahl. Die Steuerung und das Takten dieses Speichers geschieht über die Leitung 141. Über die Datenleitung 144 gelangt die gespeicherte Gütezahl auf einen weiteren Prioritätsdekodierer 80, dem entsprechend die Gütezahlen sämtlicher anderer Registergruppen zugeführt sind. Das Ausgangssignal dieses Dekodierers 80 gelangt über eine weitere Datenleitung 81 ebenfalls als zusätzliches Steuersignal auf den Multiplexer 60. Auf diese Weise ist es zusätzlich möglich, die Reihenfolge, in der die unterschiedlichen Registergruppen ausgenutzt werden, durch diese Gütezahlen festzulegen. Ist eine Sequenz in einer Registergruppe zum Beenden einer Tachykardie effektiv, so wird dabei der Zählerinhalt des Vorwärts-/Rückwärts-Zählers 140 erhöht, d.h. die Gütezahl wird vergrössert. Ist die verwundete Sequenz hingegen ineffektiv, so wird in entsprechender Weise die Gütezahl verringert.

Aus Anschaulichkeitsgründen sind in der in Figur 4 dargestellten Schaltung einer Reihe von Taktsignalen sowie einige Torschaltungen zu den verschiedenen Registern und unter Umständen auch evtl. zweckmässige Verzögerungsglieder für den zeitgerechten Funktionsablauf nicht expliziet dargestellt.

Die Schaltung gibt ein vorteilhaftes Ausführungsbeispiel eines Herzschrittmachers an. Es ist jedoch möglich, beispielsweise auf Teile wie das RR-Register oder das Register für die Gütezahl zu verzichten, ohne den Rahmen der Erfindung zu verlassen.

Eine einfache Möglichkeit zum Ausnützen der Registergruppen und zum Verändern der Registerwerte kann z.B. auch darin bestehen, dass jede neue effektive Sequenz in das erste der N Registergruppen eingespeichert wird und die vorhandenen Registerwerte jeweils in die nächste Registergruppe verschoben werden. Die in der N-ten Registergruppe gespeicherte Sequenz wird dabei gelöscht.

Ebenso sind andere Kombinationen denkbar. So ist es beispielsweise auch möglich, zumindest Teile der dargestellten Schaltung durch einen Mikroprozessor zu verwirklichen.

**Ansprüche**

1. Herzschrittmacher zum Beenden einer Tachykardie mit folgenden Verfahrensschritten :
    a) Detektieren einer Tachykardie
    b) Auslösen einer Routine zum Beenden der Tachykardie bestehend aus
        1. dem Erzeugen mindestens einer ersten Sequenz mit einer Anzahl n ($n \geq 1$) von Stimulierungsimpulsen innerhalb eines durch die möglichen Zeiten S1...Sn dieser Stimulierungsimpulse aufgespannten n-dimensionalen Raumes,
        2. dem Überwachen der Herzreaktion auf die Stimulierung,
        3. dem Verändern der Zeiten in der Sequenz und/oder der Anzahl n der Impulse derart, das zumindest ein Teil des n-dimensionalen Raumes abgerastert wird, bis eine effektive Sequenz zum Beenden der Tachykardie gefunden ist, **gekennzeichnet durch**

c) Speichern von N(N > 1) effektiven Sequenzen in N(N > 1) Registern (9, 100) eines Speichers (8, 100),

d) Verwendung der vorhandenen Registerwerte als Sequenzen für Stimulierungsimpulse bei einer erneut auftretenden Tachykardie in einer vorgebbaren Reihenfolge, an die sich, wenn alle sequenzen ineffektiv sind, erneut die Routine zum Beenden der Tachykardie anschließt und

e) Austausch einer gespeicherten Sequenz gegen eine in einer erneuten Routine gewonnen effektive Sequenz, wenn alle gespeicherten Sequenzen ineffektiv waren.

2. Herzschrittmacher nach Anspruch 1, **dadurch gekennzeichnet,** daß die gespeicherten Sequenzen in der Reihenfolge, in der sie zuletzt effektiv waren, geordnet und/oder ausgenützt werden.

3. Herzschrittmacher nach Anspruch 1, **dadurch gekennzeichnet,** daß zusätzlich zu den Sequenzen in den einzelnen Registern (9, 100) die dazugehörigen Tachykardie-Intervalle (RR) gespeichert werden und daß die Registerwerte beim erneuten Auftreten einer Tachykardie danach ausgenutzt werden, wie nahe das neue Tachykardie- Intervall an die gespeicherten Tachykardie-Intervalle herankommt.

4. Herzschrittmacher nach Anspruch 1, **dadurch gekennzeichnet,** daß jedem Register (9, 100) eine relative Gütezahl zugeordnet ist, die jedesmal erhöht wird, wenn die entsprechende Sequenz zum Beenden einer Tachykardie effektiv ist, und jedesmal verringert wird, wenn die Sequenz ineffektiv ist, und daß die einzelnen Register (9, 100) in der Reihenfolge dieser Gütezahlen ausgenutzt werden.

5. Herzschrittmacher nach einem der Ansprüche 1 bis 4 mit der Anzahl n der Stimulierungsimpulse größer als 1 (n > 1) und gleichen Intervallen zwischen den einzelnen Impulsen, **dadurch gekennzeichnet,** daß in jedem Register jeweils ein Intervall und die Anzahl n gespeichert wird.

6. Herzschrittmacher nach einem der Ansprüche 1 bis 5, **dadurch gekennzeichnet,** daß das Abrastern des n-dimensionalen Raumes derart geschieht, daß die Zeit Sn eine Funktion von S1 ist.

7. Herzschrittmacher nach Anspruch 6, **dadurch gekennzeichnet,** daß die Zeit Sn eine lineare Funktion erstere Grades von S1 ist.

8. Herzschrittmacher nach Anspruch 7, **dadurch gekennzeichnet,** daß Sn gleich S1 ist.

9. Herzschrittmacher nach einem der Ansprüche 1-8 mit mindesten einem Signaldetektor, einem steuerbaren Stimulierungsimpuls-Generator mit einstellbarem Zeitintervall zwischen den Impulsen sowie einem Tachykardiedetektor, **gekennzeichnet durch** einen Speicher (8, 100) mit N Registern (9, 100), in die jeweils mindestens die Sequenz von n Stimulierungsimpulsen eingebbar ist, die für die Beendigung einer Tachykardie effektiv war, dessen Registerwerte zum Einstellen des Generators auf die entsprechende Sequenz dient, sowie eine Steuerlogik (7, 60) für die Wahl eines Registers (9, 100) und/oder die Änderung der Registerwerte, die durch den Tachykardiedetektor (5, 30) getaktet ist.

10. Herzschrittmacher nach Anspruch 9, **dadurch gekennzeichnet,** daß der Speicherausgang auch auf die Steuerlogik (7, 60) rückgekoppelt ist.

11. Herzschrittmacher nach Anspruch 9 oder 10, **dadurch gekennzeichnet,** daß die Steuerlogik mindestens ein über die Herztätigkeit getaktetes Rechenglied (50) sowie einen Multiplexer (60) aufweist, über den die einzelnen Register (100) ausgewählt und die Zeitintervalle in diesen Registern (100) einstellbar sind.

12. Herzschrittmacher nach einem der Ansprüche 9 bis 11, **dadurch gekennzeichnet,** daß jedes Register (100) mindestens zwei Vorwärts-/Rückwärts-Zähler (120, 130) aufweist, von denen der eine zum Speichern des Zeitintervalles S1 und der andere zum Speichern des Zeitintervalles S2 dient.

13. Herzschrittmacher nach einem der Ansprüche 9 bis 12, **dadurch gekennzeichnet,** daß zumindest Teile des Tachykardiedetektors (5, 30) oder der Steuerlogik (7, 50, 60) oder des Speichers (8, 100) oder Teile von diesen durch einen Mikroprozessor gebildet sind.

**Claims**

1. Heart pacemaker for arresting a tachycardia by means of the following process steps :

a) detection of a tachycardia

b) initiation of a routine for arresting the tachycardia, consisting of

1. the generation of at least one first sequence having a number n (n ≥ 1) of stimulating pulses within an n-dimensional space spanned by the possible times S1...Sn of said stimulating pulses,

2. monitoring the reaction of the heart to the stimulation,

3. variation of the times in the sequence and/or the number n of the pulses in such a way that at least a part of the n-dimensional space is scanned until an effective sequence for arresting the tachycardia is found, characterised by

c) storage of N (N > 1) effective sequences in N (N > 1) registers (9, 100) of a memory (8, 100),

d) use of the available register values as sequences for stimulating pulses, in the event of a renewed occurrence of tachycardia, in a prescribed order of succession which is followed once again, if all sequences are ineffective, by the routine for arresting the tachycardia, and

e) exchange of a stored sequence for an effective sequence obtained in a renewed routine, if all stored sequences were ineffective.

2. Heart pacemaker according to Claim 1, characterised in that the stored sequences are ordered and/or utilised in the order of succession in which they were last effective.

3. Heart pacemaker according to Claim 1, characterised in that in addition to the sequences the associated tachycardia intervals (RR) are stored in the individual registers (9, 100), and in that in the event of a renewed occurrence of tachycardia the register values are utilised in accordance with how near the new tachycardia interval comes to the stored tachycardia intervals.

4. Heart pacemaker according to Claim 1, characterised in that each register (9, 100) is assigned a relative figure of merit which is increased each time that the corresponding sequence is effective for arresting a tachycardia, and is reduced each time that the sequence is ineffective, and in that the individual registers (9, 100) are utilised in the order of succession of said figures of merit.

5. Heart pacemaker according to one of Claims 1 to 4 with the number n of the stimulating pulses larger than 1 (n > 1) and equal intervals between the individual pulses, characterised in that in each case one interval and the number n are stored in each register.

6. Heart pacemaker according to one of Claim 1 to 5, characterised in that the scanning of the n-dimensional space takes place in such a way that the time Sn is a function of S1.

7. Heart pacemaker according to Claim 6, characterised in that the time Sn is a linear function of the first degree in S1.

8. Heart pacemaker according to Claim 7, characterised in that Sn is equal to S1.

9. Heart pacemaker according to one of Claim 1-8 with at least one signal detector, a controllable stimulating pulse generator with a time interval adjustable between the pulses, as well as a tachycardia detector, characterised by a memory (8, 100) with N registers (9, 100) into which it is possible to input in each case at least the sequence of n stimulating pulses that was effective for arresting a tachycardia, the register values of which serve to set the generator to the corresponding sequence, as well as a control logic (7, 60) for selecting a register (9, 100) and/or the change in the register values which is clocked by the tachycardia detector (5, 30).

10. Heart pacemaker according to Claim 9, characterised in that the memory output is fed back to the control logic (7, 60).

11. Heart pacemaker according to Claim 9 or 10, characterised in that the control logic has at least one arithmetic element (50) clocked via the cardiac activity as well as a multiplexer (60) via which the individual registers (100) are selected and the time intervals can be set in said registers (100).

12. Heart pacemaker according to one of Claims 9 to 11, characterised in that each register (100) has at least two up-down counters (120, 130), of which one serves to store the time interval S1 and the other to store the time interval S2.

13. Heart pacemaker according to one of Claims 9 to 12, characterised in that at least parts of the tachycardia detector (5, 30) or of the control logic (7, 50, 60) or of the memory (8, 100) or parts of these are formed by a microprocessor.

## Revendications

1. Stimulateur cardiaque servant à arrêter une tachycardie, moyennant la mise en oeuvre des phases opératoires suivantes :

    a) détection d'une tachycardie,

    b) déclenchement d'un sous-programme pour arrêter la tachycardie et consistant en

        1. la production d'au moins une première séquence possédant un nombre n (n ≥ 1) d'impulsions de stimulation à l'intérieur d'un espace n-dimensionnel occupé par les durées possibles S1...Sn de ces impulsions de stimulation,

        2. le contôle de la réaction du coeur à la stimulation,

        3. la modification des durées de la séquence et/ou du nombre n des impulsions de telle sorte qu'au moins une partie de l'espace n-dimensionnel est explorée sous la forme d'une trame jusqu'à ce qu'une séquence efficace pour arrêter la tachycardie soit trouvée, caractérisé par

    c) la mémorisation de N (N > 1) séquences efficaces dans N (N > 1) registres (9,100) d'une mémoire (8, 100),

    d) l'utilisation des valeurs présentes, situées dans les registres, en tant que séquences pour des impulsions de stimulation lors de l'apparition réitérée d'une tachycardie, selon une suite pouvant être prédéterminée à laquelle succède à nouveau le sous-programme permettant d'arrêter la tachycardie, lorsque toutes les séquences sont inefficaces, et

    e) le remplacement d'une séquence mémorisé par une séquence efficace obtenue dans un nouveau sous-programme lorsque toutes les séquences mémorisées ont été inefficaces.

2. Stimulateur cardiaque suivant la revendication 1, caractérisé par le fait que les séquences mémorisées sont rangées et/ou utilisées dans l'ordre, dans lequel elles ont été efficaces en dernier lieu.

3. Stimulateur cardiaque suivant la revendication 1, caractérisé par le fait qu'en plus des séquences, les intervalles associés de tachycardie (RR) sont mémorisés dans les différents registres (9, 100) et que les valeurs des registres sont utilisées lors de l'apparition réitérée d'une tachycardie en fonction du degré de proximité du nouvel intervalle de tachycardie par rapport aux intervalles de tachycardie mémorisés.

4. Stimulateur cardiaque suivant la revendication 1, caractérisé par le fait qu'à chaque registre (9, 100) est associé un indice de qualité relatif qui est accru chaque fois que la séquence correspondante est efficace pour arrêter une tachycardie, et est réduit chaque fois que la séquence est inefficace, et que les différents registres (9, 100) mont utilisés dans l'ordre de ces indices de qualité.

5. Stimulateur cardiaque suivant l'une des revendications 1 à 4, avec le nombre n dos impulsions de stimulation supérieur à 1 (n > 1), et des intervalles identiques entre les différentes impulsions, caractérisé par le fait que respectivement un intervalle et le nombre n sont mémorisés dans chaque registre.

6. Stimulateur cardiaque suivant l'une des revendications 1 à 5, caractérisé par le fait que l'exploration de l'espace n-dimensionnel s'effectue grâce au fait que la durée Sn est fonction de S1.

7. Stimulateur cardiaque suivant la revendication 6, caractérisé par le fait que la durée Sn est une fonction linéaire du premier degré de S1.

8. Stimulateur cardiaque suivant la revendication 7, caractérisé par le fait que Sn est égal à S1.

9. Stimulateur cardiaque suivant l'une des revendications 1-8, comportant au moins un détecteur de signaux, un générataur commandable d'impulsions de stimulation, l'intervalle de temps entre les impulsions étant réglable, ainsi qu'un détecteur de tachycardie, caractérisé par une mémoire (8, 100) comportant N registres (9, 100), dans lesquels peut être introduite respectivement au moins la séquence de n impulsions de stimulation, qui était efficace pour l'arrêt d'une tachycardie, et dont les valeurs, situées dans les registres, servent à régler le générateur sur la séquence correspondante, ainsi qu'un circuit logique de commande (7, 60) qui est utilisé pour sélectionner un registre (9, 100) et/ou modifier les valeurs des registres et qui est commandé de façon cadencée par le détecteur de tachycardie (5, 30).

10. Stimulateur cardiaque suivant la revendication 9, caractérisé par le fait que la sortie de la mémoire est également couplée par réaction au circuit logique de commande (7, 60).

11. Stimulateur cardiaque suivant la revendication 9 ou 10, caractérisé par le fait que le circuit logique du commande comporte au moins un circuit de calcul (50) commandé de façon cadencée par l'intermédiaire de l'activité cardiaque, ains qu'un multiplexeur (60), au moyen duquel les différents registres (100) sont sélectionnés et au moyen duquel les intervalles de temps dans ces registres (100) peuvent être réglés.

12. Stimulateur cardiaque suivat l'une des revendications 9 à 11, caractérisé par le fait que chaque registre (100) comporte au moins deux compteurs progressifs/ régressifs (120, 130), dont l'un sert à mémoriser l'intervalle de temps S1 et dont l'autre sert à mémoriser l'intervalle de temps S2.

13. Stimulateur cardiaque suivant l'une des revendications 9 à 12, caractérisé par la faut qu'au moins des parties du détecteur de tachycardie (5, 30) ou du circuit logique de commande (7, 50, 60) ou de la mémoire (8, 100) ou de parties de cette dernière sont formés par un microprocesseur.

FIG 1

FIG 2

# FIG 3

FIG 4